# EUROPEAN PATENT APPLICATION

(11) **EP 4 748 300 A1**
(43) Date of publication of application: **27.05.2026**
(21) Application number: 24841843.6
(22) Date of filing: 06.06.2024
(51) Int. Cl.: A61B 5/02, A61B 5/1172, G06V 40/12, G06V 40/13

(54) **PORTABLE BIOMETRIC READER FOR CHILDREN AND NEWBORNS**

(30) Priority: 18.07.2023 BR 102023014432
(71) Applicant: Natosafe Tecnologia da Informação S/A, 80250-104 Curitiba/PR (BR)
(72) Inventor: MACIOSKI, Renata, 83015-065 São José dos Pinhais/PR (BR); ZANLORENZI, Jeronymo Gustavo, 83040-320 São José dos Pinhais/PR (BR); WASILEVSKI, Ildefonso, 81900-470 Curitiba/PR (BR); FILIPAK, Marcelo, 80530-320 Curitiba/PR (BR)
(74) Representative: Torner, Juncosa I Associats, SL
(86) International application number: PCT/BR2024/050235
(87) International publication number: WO 2025/015395

(57) **Abstract**

The present patent of invention describes a portable biometric reader for children and newborns that, in accordance with the features thereof, enables the formation of a biometric reader (1) having its own specific portable, ergonomic electronic fingerprinting structure based on a structural body (2), a high-resolution optical fingerprinting sensor (8) with integrated sensor, lenses, prism, illumination and USB interface, and a posterior internal female USB-C adaptor (9), with the aim of enabling complete optimization of the procedures of reading and capturing in real time images of the fingerprints of the fingers of children and newborns by collecting images of a sufficient quality to optimize differentiation of the dimensional characteristics of valleys, ridges and details that are very small, as well as having dimensions, a weight and a design that are suitable for easy handling in small spaces, such as in maternity wards

## Description

The present patent of invention refers to fingerprint readers in general, more specifically to a portable biometric reader for children and newborns which, in accordance with the features thereof, has as its basic principle the formation of a biometric reader in its own specific portable, ergonomic electronic fingerprinting structure based on a structuring body, a high-resolution optical fingerprint sensor with an integrated sensor, lenses, prism, illumination and a USB interface, and a posterior internal female USB-C adapter, so as to enable, in a practical, safe and accurate way, complete optimization of the procedures for reading and capturing real-time fingerprint images of children's and newborns' fingers by collecting high-quality images to optimize differentiation of the dimensional characteristics of valleys, ridges and details that are very small, as well as having dimensions, a weight and a design that are suitable for easy handling in small spaces, such as in maternity wards.

The biometric reader has a specific design and format and easy access for better adaptation and safety of users, is practical and functional, very affordable, and, due to its overall characteristics and dimensions, easily adaptable to a wide range of children and newborns, places and users in general, regardless of their characteristics.

Biometrics is any methodology or technology that enables a person to be identified directly from their physical characteristics. As no two people are exactly the same, analyzing unique, non-transferable features has become a common solution in situations requiring individual recognition. Biometrics is one of the safest ways to identify individuals and protect data.

Biometrics is the application of metrics to biological attributes for the purpose of identifying and verifying individuals; its operation involves capturing and analyzing biometric data, such as facial recognition, fingerprinting, or iris scanning, and this data is then compared with a database to identify the user.

Fingerprinting is the oldest and most cost-effective method of biometric recognition and is extremely reliable given the exceptionally low mutability of data over time, having migrated smoothly from analog to digital means. As fingerprints do not change throughout life, the only way for problems to occur is for a person to lose their fingerprints. Regardless of the reason, this method continues to be used, on its own or in combination with others.

A fingerprint is made up of small elevations (papillae) in the skin on the fingertips that form sets of unique patterns, and these patterns are not repeated on other fingers or in other people, which is why fingerprints can and are used to identify individuals; in other words, our fingerprints are unique: no two people have the same ones.

The biometric reader has a sensor that captures people's fingerprints and sends them to a control system, which already stores other information about them. It works by enabling the reader to capture fingerprints by positioning fingers on it, and these are then compared with the ones registered in the database for identification.

The use of papilloscopic images is more appealing than that of other biometrics because they are non-invasive, easy to apply, and accepted by a variety of agencies, in addition to the significant advantage of immutability, making them ideal for future identification purposes, particularly in registration systems.

However, the current identification systems for children and newborns are very outdated, as they lack the necessary devices to identify them properly and almost making it impossible for a safe and practical identification, mainly because collecting fingerprints from children and newborns is a difficult process as their papillary ridges are, on average, 2.5 to 3 times smaller than those of adults, and they are more susceptible to deformation.

More specifically, fingerprints of children and newborns up to five years of age cannot be recorded with the accuracy required by conventional 500dpi devices, as the dimensions of the valleys, ridges and details are much smaller compared to those of young people and adults.

Currently, commercially available biometric systems are used solely for identifying adults. Despite the evolution of biometric technologies, few approaches have been developed to identify children and newborns. This means disregarding the millions of births that occur worldwide each year. The problem becomes even more significant when we consider the vast number of children worldwide between the ages of zero and five.

Therefore, the efficiency of fingerprint registration and identification systems depends directly on the quality of the captured images; that is, the captured images must meet certain resolution and sharpness standards. The standards for certified biometric readers for identification must include a minimum resolution for fingerprint registration. Although these resolution values are sufficient for capturing good images and resolving the details of adult fingerprints, the same cannot be said for children and newborns, whose fingerprints and details are significantly smaller than those of adults.

It should be noted that, in the context of digital biometric readers, there are many pieces of equipment, but most of these are based on an image generation method (FTIR or scattering) and an optical architecture that incorporates special components designed to reduce distortion and improve image quality. In other words, no other equipment allows the application in reading and capturing images of fingerprints from children and newborns with high resolution and a minimum area for acquisition, as well as being highly portable and easy to handle.

In this context, the invention of devices capable of generating high-quality, accurate fingerprint images of children and newborns, primarily at the time of birth, has become essential for families and government agencies; it represents a significant step in the identification of children and newborns, enabling their effective medical registration and ensuring their safety.

A broad analysis of the literature was conducted to establish the current state of the art regarding biometric readers, specifically portable biometric readers for children and newborns, the object of this patent, and only one document relevant to the current state of the art was found that relates to the specific object claimed in this patent, namely a portable biometric reader that can be held with one hand and has the minimum resolution necessary for acquiring fingerprint images of children and newborns, more specifically the patent of invention BR IO 2019 016898 6 - titled "High Resolution Portable Biometric Reader for Newborns', filed on August 14, 2019 and having as holders Akiyama *- Industria e Comércio de Equipamentos Eletrônicos e Sistemas,* and SENAI - *Serviço Nacional de Aprendizado Industrial.*

This high-resolution, portable biometric reader for newborns has proven capable of collecting images of sufficient quality to differentiate the essential features; however, as it is also used to capture biometrics from palms and soles, its dimensions, weight and ergonomics make it unsuitable for use in small enclosures such as maternity wards, particularly when handling newborns in incubators.

Another issue relates to the equipment's robustness, which requires constant, periodic maintenance and manual cleaning of the prism interior, lens focus adjustment, and calibration or recalibration of the sensor camera, mainly because the capture set comprises components such as the sensor, lenses, prism, illumination and power and data transfer interfaces, which are not integrated into a single device.

Additionally, it should be noted that, although the area covered by the collected image is approximately twenty percent of the total sensor area and the image is high resolution and definition, these characteristics together do not provide sufficient quality for analysis and processing by papilloscopist experts and algorithms, because higher-quality images of collected fingerprints optimize the differentiation of the details from fingerprints and dirt/finger varnish for children and newborns.

Finally, this equipment has proprietary driver requirements and is not compatible with the USB Video Class (UVC) driver, not fitting as a plug-and-play device, and requiring the installation of a specific SDK and driver to operate perfectly as an image collection instrument.

In this context, the overall design of this portable biometric reader for children and newborns, object of the present patent, is based entirely on its simple and robust structuring with a minimum of components and extremely simplified, safe and optimized operation, combined with practical manufacturing and maintenance procedures, in order to generate a biometric reader that is practical and efficient, capable of collecting high-quality fingerprint images of children and newborns to differentiate the essential features, dimensions of the valleys, ridges, and details thereof, which are much smaller in newborns and children up to five years of age, compared to young people and adults, mainly because it is only used to capture biometrics from the fingers, so that its dimensions, weight, design, ergonomics and portability make it suitable to be easily held with one hand in small spaces, such as maternity wards, particularly when handling newborns in incubators.

More specifically, the biometric reader is a single device that integrates a sensor, lenses, a prism, illumination, and USB interfaces, so there is no need for periodic maintenance such as camera recalibration, lens focus adjustment, and prism interior cleaning. In addition to these components being lighter, more economical, and smaller, the optical system with a monochrome image sensor provides an image resolution of 5080dpi and a prism capture area of 18mm x 22mm, plus the area used by the captured/collected image is approximately seventy percent of the total sensor area.

It should be highlighted that these characteristics provide high-quality, high-resolution images that optimize the differentiation of the details from fingerprints and dirt on children's and newborns' fingers, enabling higher quality analysis and processing by papilloscopist experts and fingerprint algorithms, besides generating a real-time fingerprint image data stream for developers through the USB interface.

The patent in question is characterized by bringing together components and processes in a differentiated design to meet the various requirements the nature of its use demands, i.e., the acquisition of biometrics from children and newborns. This design guarantees a biometric reader that is highly efficient, functional, resistant, durable, safe, versatile, accurate, and economical, due to the excellent technical qualities added, which provide advantages and improvements in the routine procedures for registering the details of children and newborns, and whose general characteristics differ from other forms and models widely known in the current state of the art.

The present patent consists of a modern, efficient, safe and functional portable biometric reader for children and newborns formed from a set of properly incorporated electronic and biometric solutions, comprising a complete and differentiated biometric reader with an exclusive design and optimal finish details, as well as its own unique characteristics, which incorporates its own and specific electronic structure that is highly durable and resistant, ergonomically shaped, and containing a perfectly integrated, symmetrically arranged structuring body with a front cover and internal brackets for a USB-C adapter and an optical sensor as a manual support element for the operator; an optical fingerprint sensor as an element for reading and capturing fingerprints; and a female USB-C adapter for power supply and as a transferring element for collected data, in order to enable the formation of a single, complete and safe set, whose internal and external forms and arrangements enable its perfect adaptation to children and newborns of all types, being specially designed for this purpose with its geometry.

The present biometric reader is based on the application of components and processes in a differentiated design, without, however, achieving a high degree of sophistication or complexity, making it possible to solve some of the main drawbacks of other known forms and models in the current state of the art which are used for capturing fingerprints of children and newborns, which are located in a work range in which the difficulties in use and application, low efficiency and performance, and accidents are frequent and the forms and/or models are based on simple adaptations and are therefore highly insecure, prone to deterioration and fragility, and have little durability and resistance, low versatility, high inaccuracy, are laborious to apply, prone to high losses, lacking in ergonomics and performance, expensive, high overall volume and weight, complex handling, high maintenance, resulting in a significant waste of time.

The objectives, advantages, and other important characteristics of the patent in question can be more easily understood when read together with the attached figures, in which:
Figure 1A shows a top perspective view of the portable biometric reader for children and newborns.
Figure 1B shows a bottom perspective view of the portable biometric reader for children and newborns.
Figure 1C shows a right-side view of the portable biometric reader for children and newborns.
Figure 1D shows a left side view of the portable biometric reader for children and newborns.
Figure 1E shows a top view of the portable biometric reader for children and newborns.
Figure 1F shows a bottom view of the portable biometric reader for children and newborns.
Figure 1G shows a front view of the portable biometric reader for children and newborns.
Figure 1H shows a rear view of the portable biometric reader for children and newborns.
Figure 1I shows a perspective view of the internal USB-C adapter bracket of the portable biometric reader for children and newborns.
Figure 1J shows a perspective view of the internal support for the optical sensor of the portable biometric reader for children and newborns.

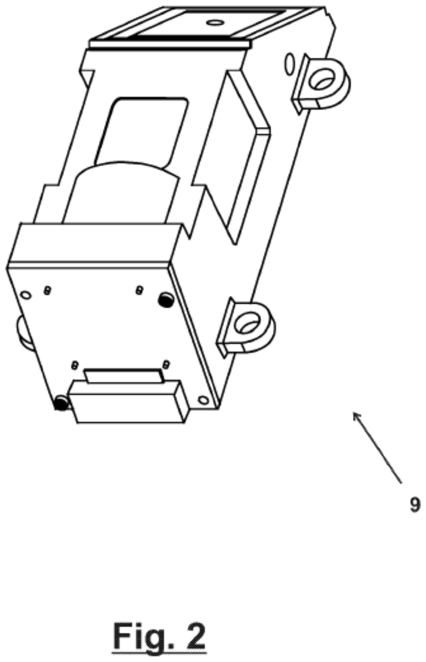

Figure 2 shows a perspective view of the fingerprint sensor on the portable biometric reader for children and newborns.

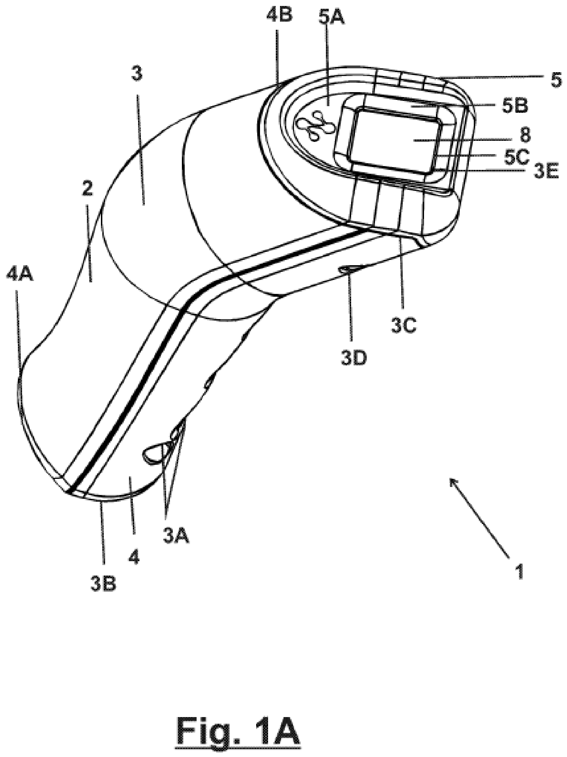

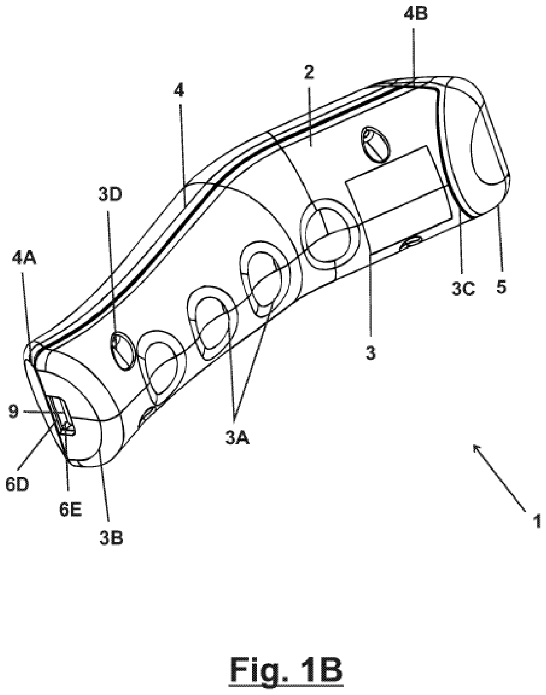

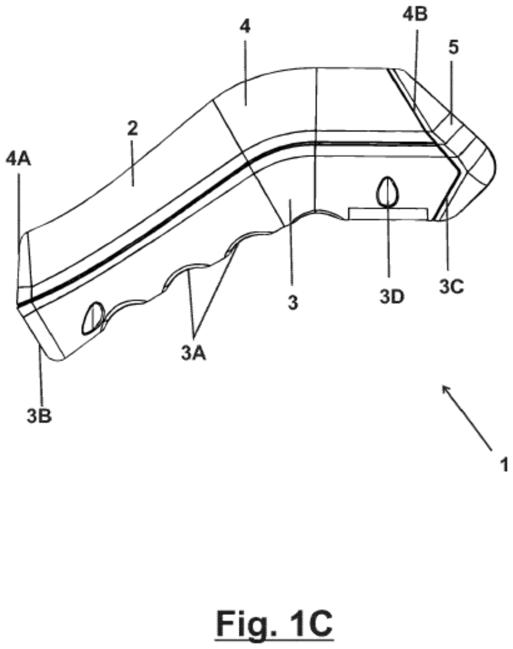

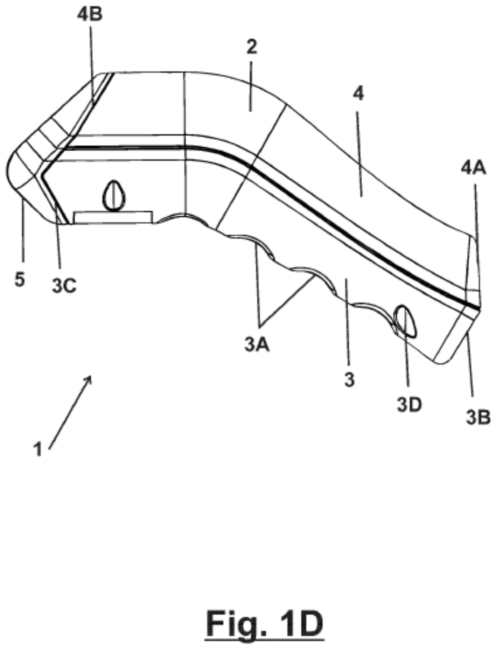

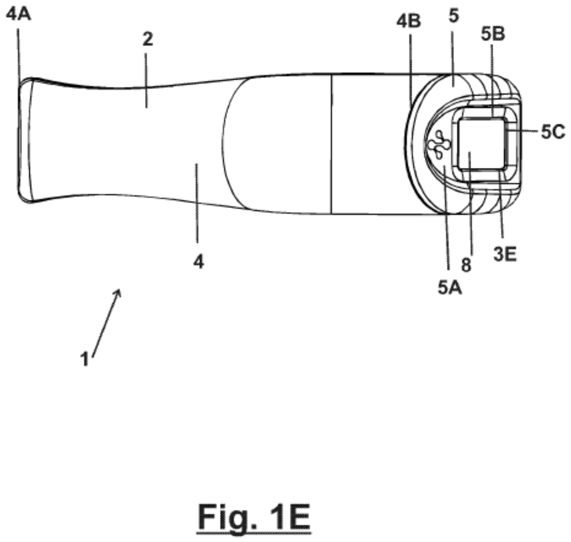

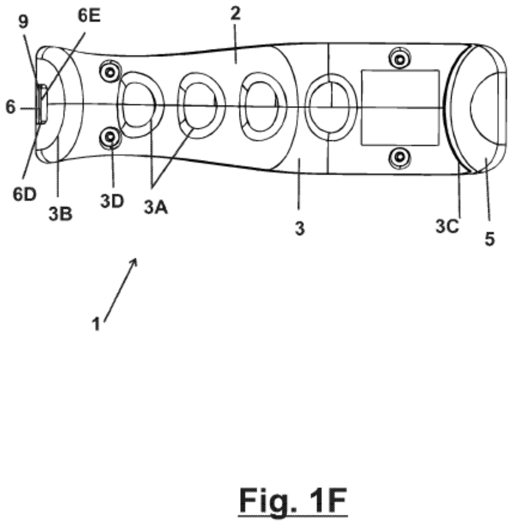

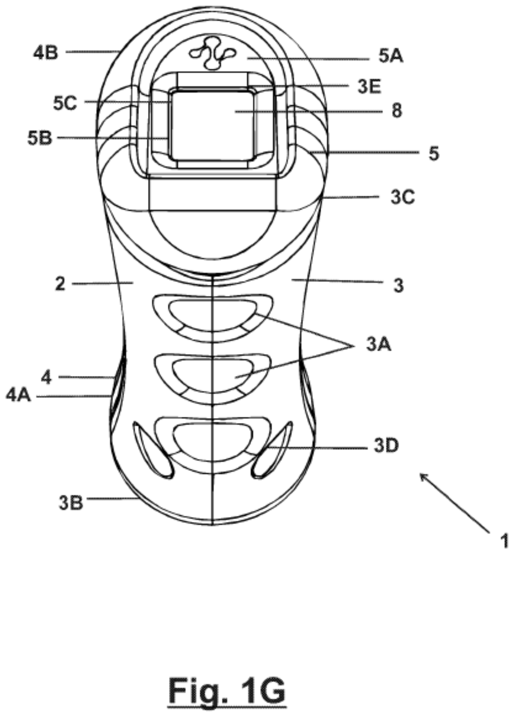

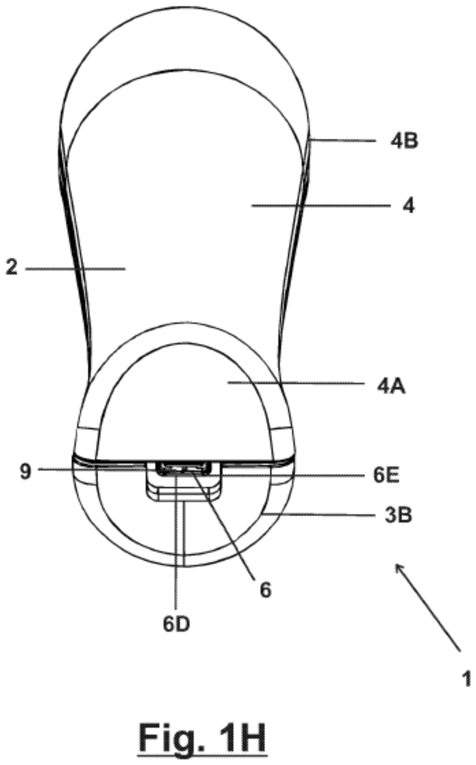

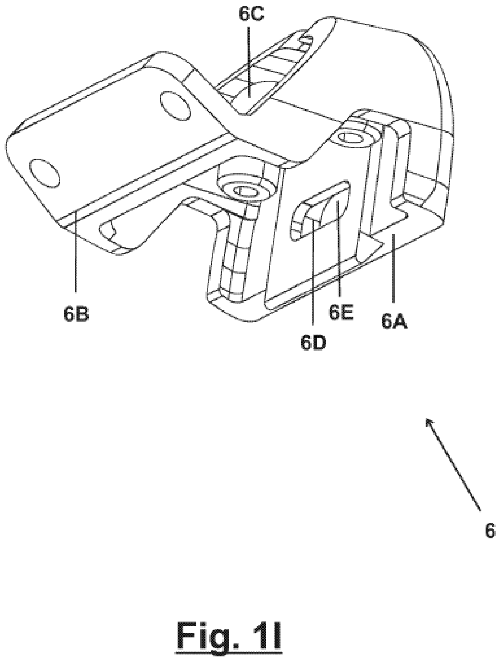

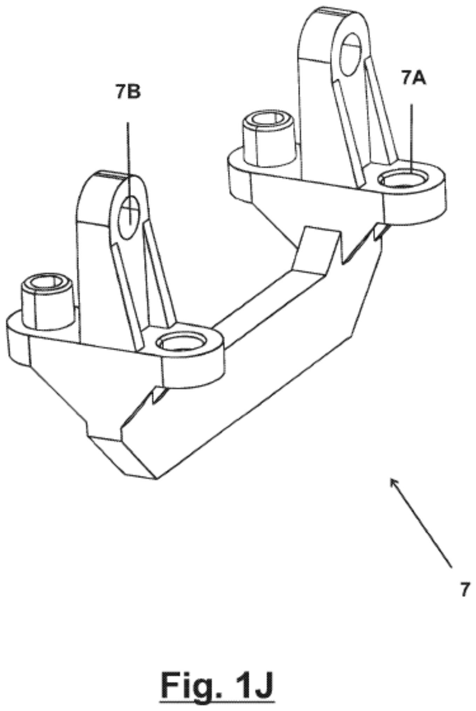

As can be inferred from the attached figures, which illustrate and integrate this descriptive report of the patent of invention for a "Portable Biometric Reader for Children and Newborns", Figure 1A provides a general overview of the complete biometric reader (1) with its own characteristics, which incorporates its own and specific electronic structure of high durability and resistance, and that has an ergonomic shape and internal and external arrangements that adapt to children and newborns of all types, containing perfectly integrated and symmetrically arranged among themselves:
- a curvilinear structuring body (2) with curved edges that is arranged symmetrically along the entire length of the biometric reader (1) comprising:
   - a curvilinear base (3) with a concave cross-section, arranged symmetrically along the lower length of the structuring body (2) and having curvilinear indentations (3A) (for positioning and supporting a user's fingers) and arranged parallel, symmetrically and spaced along the lower face of the base (3); a rectilinear rear chamfer (3B) arranged parallel and symmetrically along the entire length of the rear face of the base (3); a front chamfer (3C) similar to a lying-down "U" and arranged parallel and symmetrically along the entire length of the front face of the base (3); a rectangular rear opening (3D) arranged parallel and symmetrically centered at the supero posterior end of the rear face of the base (3); a plurality of cylindrical openings (3E) arranged perpendicular and symmetrically spaced at the inferolateral ends of the base (3); and a plurality of cylindrical and semi-cylindrical internal fins and supports arranged parallel and spaced on the inside of the base (3);
   - a curvilinear cover (4) with a concave cross-section, arranged parallel and symmetrically aligned and overlapping along the entire length of the upper face of the base (3) and the entire upper length of the structuring body (2) and having a rectilinear rear chamfer (4A) parallel and symmetrically arranged along the entire length of the rear face of the cover, and a rectilinear front chamfer (4B) parallel and symmetrically arranged along the entire length of the rear face of the cover (4), a concave rear opening parallel and symmetrically arranged along the entire length of the rear face of the cover and a plurality of cylindrical and semi-cylindrical internal fins and supports symmetrically aligned with the internal fins and supports of the inner face of the base (3);
   - a curvilinear front frame (5) with a cross-section similar to an "L", arranged parallel and symmetrically along the entire length of the rear faces to the base (3) and cover (4), and to the rear face of the structuring body (2), and having a semicircular main rear recess (5A) with curved ends arranged parallel and symmetrically along the entire length of the supero anterior face of the front frame (5); a rectangular secondary front recess (5B) with curved ends arranged parallel and symmetrically centered at the bottom of the main rear recess (5A); and a rectangular front opening (5C) arranged parallel and symmetrically along the entire length of the secondary front recess (5B);
   - a semi-cylindrical internal USB-C adapter bracket (6) symmetrically arranged in a segment of the postero interior part of the structuring body (2) and having a semicylindrical front base (6A) vertically and symmetrically arranged in the front part of the internal USB-C adapter bracket (6); a rear flap (6B) similar to an "L" arranged parallel and symmetrically aligned at the rear part of the front base (6A); a rear semicircular opening (6C) arranged parallel and symmetrically on the rear flap (6B); a parallelepipedal front recess (6D) arranged parallel and symmetrically centered at the anteroinferior part of the front base (6A); a rectangular front opening (6E) arranged parallel and symmetrically centered on the front recess (6D); and
   - an internal support for the optical sensor (7) similar to a "U" arranged perpendicular and symmetrically in the anterointernal part of the structuring body (2) and having a plurality of cylindrical vertical openings (7A) arranged vertically at the lateral ends of the lower parts of the internal support for the optical sensor (7); and a plurality of cylindrical horizontal openings (7B) horizontally arranged at the upper ends of the upper parts of the internal support for the optical sensor (7);
   - a parallelepipedal optical fingerprint sensor (8) symmetrically arranged along the anterointernal part of the structuring body (2), passing through the internal support for the optical sensor (7) and in the rear alignment of the front opening (5C), as a fully touch-reflective single-module optical structure CMOS imaging camera and integrated by an optical sensor, lenses, a prism, a LED light for illumination, USB Video Class (UVC) video interface with a USB converter board and a power supply; and
   - a USB-C female adapter (9) symmetrically centered at the front opening (6E) of the internal USB-C adapter bracket (6) and interconnected to the USB Video Class (UVC) video interface with a USB converter board.

The components of the biometric reader (1) fit together and lock perfectly inside its structuring body (2), forming a single module with a fully ergonomic shape and design, that is, the internal USB-C adapter bracket (6) and the internal support for the optical sensor (7) fit and attach to the inner part of the base (3), as well as the optical fingerprint sensor (8) to the internal support of the optical sensor (7), the front frame (5) to the front end of the base (3) and the cover (4) over the entire upper length of the base (3), and the upper end of the front frame (5) locks and closes all the components.

The biometric reader (1) captures and reads fingerprint images of children's and newborns' fingers through the use of the optical fingerprint sensor (8) and, consequently, transfers these fingerprints in real time to a wide range of mobile devices and personal computers via its USB Video interface (Class UVC) and USB converter board. This same USB Video Class (UVC) video interface with a USB converter board also enables the optical fingerprint sensor (8) to be charged.

Similarly, the biometric reader (1), by making use of its optical fingerprint sensor (8), such as a CMOS image camera with a fully touch-reflective single-module optical structure that integrates an optical sensor, lenses, a prism, an illumination system, and a USB video interface with a converter board, enables an optical system with a monochrome image sensor that provides an image with a resolution of 5080dpi and a prism with a capture area of 18 mm x 22 mm that covers seventy percent of the total sensor area.

The biometric reader for children and newborns offers the following specific advantages: automatic LED light control, automatic activation of the touch sensor and redefinition of the optical fingerprint sensor (8); no need for a direct connection of the optical fingerprint sensor (8) to the host device thanks to the USB Video Class (UVC) video interface and USB converter board; can be used as an image collection instrument without the need to install specific SDKs and drivers, which can lead to the development of SDKs and computational methods for fingerprint recognition by third parties; can capture real-time fingerprint images of children and newborns via the USB Type C interface compatible with USB Video Class (UVC) video drivers for connection to mobile devices and personal computers, making it a plug-and-play device; and can be applied as an image collection instrument without the need to install specific SDKs and drivers, which can lead to the development of SDKs and computational methods for fingerprint recognition by third parties.

The portable biometric reader for children and newborns has fully integrated components, nothing comes off, or breaks or bends, thus achieving a high level of performance and efficiency, combined with high durability and absolute security. Once fully integrated, the components are firmly attached to each other, thus preventing them from coming loose during use, making the set ready for use in reading and capturing the fingerprints of children and, primarily, newborns. This means the biometric reader can be used without any concerns, particularly with regard to the durability and security of its components and the safety of its users.

From all of the above, the biometric reader will be well received by hospitals and government health and security agencies, as the portable biometric reader for children and newborns has numerous advantages: safety, reliability and ease of application; high performance and yield due to its design; high levels of comfort, convenience, and safety for users; very high overall strength and durability, combined with low or no wear and tear of the set as a whole; affordable costs, providing an excellent cost/benefit ratio; practical and safe for any user; large range; requires very little general maintenance; perfect and direct adaptation to the most diverse types newborns in general; high operational accuracy; fully compatible weight and overall dimensions; high operating ergonomics; complete hygiene; seamless integration with computer systems; and the certainty of having a biometric reader that fully meets the current legislation, standards and the basic conditions necessary for its application.

These attributes enable the portable biometric reader for children and newborns to be classified as a highly versatile, efficient, practical and safe means of reading and capturing fingerprints of children and newborns of all types, in all kinds of places and by all kinds of users, regardless of their characteristics, being also very easy to apply and handle, combined with great performance and excellent overall characteristics; however, it is not limited, at any moment, to the representations described herein and must be understood in the broadest sense, that is to say, it is not limited to the specific form revealed and other similar forms are also included within the claimed scope.

## Claims

1. "PORTABLE BIOMETRIC READER FOR CHILDREN AND NEWBORNS", **characterized in that** it comprises a biometric reader (1) that incorporates an electronic structure with an ergonomic shape and containing symmetrically integrated:
- a curvilinear structuring body (2) with curved edges, arranged symmetrically along the entire length of the biometric reader (1) and comprising:
- a curvilinear base (3) with a concave cross-section, arranged symmetrically along the lower length of the structuring body (2) and having curvilinear indentations (3A) parallel and symmetrically spaced along the lower face of the base (3); a rectilinear rear chamfer (3B) arranged parallel and symmetrically along the entire length of the rear face of the base (3); a front chamfer (3C) similar to a lying down "v" arranged parallel and symmetrically along the entire length of the front face of the base (3); a rectangular rear opening (3D) parallel and symmetrically centered at the supero posterior end of the rear face of the base (3); a plurality of cylindrical openings (3E) perpendicular and symmetrically spaced at the inferolateral ends of the base (3); and a plurality of cylindrical and semicylindrical internal fins and supports arranged parallel and spaced on the inside of the base (3);
- a curvilinear cover (4) with a concave cross-section, parallel and symmetrically aligned and overlapping along the entire length of the upper face of the base (3) and the entire upper length of the structuring body (2) and having a rectilinear rear chamfer (4A) arranged parallel and symmetrically along the entire length of the rear face of the cover (4); a rectilinear front chamfer (4B) arranged parallel and symmetrically along the entire length of the front face of the cover (4), a concave front opening arranged parallel and symmetrically along the entire length of the front face of the cover (4); and a plurality of cylindrical and semi-cylindrical fins and supports arranged symmetrically aligned with the fins and internal supports of the internal part of the base (3);
- a curvilinear front frame (5) with a cross-section similar to an "L", arranged parallel and symmetrically along the entire length of the front faces to the base (3) and cover (4), and to the front face of the structuring body (2), and having a semicircular main front recess (5A) with curved ends arranged parallel and symmetrically along the entire length of the supero anterior surface of the front frame (5); a secondary rectangular front recess (5B) with curved ends arranged parallel and symmetrically centered at the bottom of the main front recess (5A); and a rectangular front opening (5C) arranged parallel and symmetrically along the entire length of the secondary front recess (5B);
- a semicylindrical internal USB-C adapter bracket (6) arranged symmetrically in a segment of the postero interior part of the structuring body (2) and having a semi-cylindrical front base (6A) arranged vertically and symmetrically on the front part of the internal USB-C adapter bracket (6); a rear flap (6B) similar to an "L" and arranged parallel and symmetrically aligned at the back of the front base (6A); a semicircular rear opening (6C) arranged parallel and symmetrically on the rear flap (6B); a parallelepipedal front recess (6D) arranged parallel and symmetrically centered in the anteroinferior part of the front base (6A); and a rectangular front opening (6E) arranged parallel and symmetrically centered in the front recess (6D); and
- an internal support for the optical sensor (7) similar to a "U" arranged perpendicularly and symmetrically in the antero interior part of the structuring body (2) and having a plurality of cylindrical vertical openings (7A) arranged vertically at the lateral ends of the lower parts of the internal support for the optical sensor (7); and a plurality of cylindrical horizontal openings (7B) arranged horizontally at the upper ends of the upper parts of the internal support for the optical sensor (7);
- a parallelepipedal optical fingerprint sensor (8) arranged symmetrically along the anterointernal part of the structuring body (2), passing through the internal support for the optical sensor (7) and in the rear alignment of the front opening (5C) as a fully touch-reflective single-module optical structure CMOS imaging camera and integrated by an optical sensor, lenses, a prism, a LED light for illumination, USB Video Class (UVC) video interface with a USB converter board and a power supply; and
- a female USB-C adapter (9) arranged symmetrically centered in the front opening (6E) of the internal USB-C adapter bracket (6) and interconnected to the USB Video Class (UVC) video interface with a USB converter board.
